# EUROPEAN PATENT APPLICATION

(11) **EP 1 847 172 A1**
(43) Date of publication of application: **24.10.2007**
(21) Application number: 06713205.0
(22) Date of filing: 07.02.2006
(51) Int. Cl.: A01K 1/00

(54) **SKIN CLEANSER COMPOSITION**

(30) Priority: 07.02.2005 JP 2005030293
(71) Applicant: Shiseido Company, Limited, Chuo-ku Tokyo 104-8010 (JP); Nalco Company, Naperville, IL 60563-1198 (US)
(72) Inventor: KIMURA, Tomohiko, SHISEIDO RESEARCH CENTER, Yokohama-shi, Kanagawa 224-8558 (JP); NODA, Akira, SHISEIDO RESEARCH CENTER, Yokohama-shi, Kanagawa 224-8558 (JP); ADACHI, Kentaro, SHISEIDO RESEARCH CENTER, Yokohama-shi, Kanagawa 224-8558 (JP); CARLSON, Wayne, M, NALCO COMPANY, Naperville, Illinois 60563-1198 (US)
(74) Representative: Henkel, Feiler & Hänzel
(86) International application number: PCT/JP2006/302062
(87) International publication number: WO 2006/087939

(57) **Abstract**

To provide a skin cleanser having superior lather quality at cleansing and improved moist and soft feeling after cleansing.

A skin cleansing composition comprising an amphoteric terpolymer which consists of; 5 to 15 mass % of an anionic monomer (ex. acrylic acid), 15 to 40 mass % of a cationic monomer (ex. dimethyldiallylammonium chloride) and 45 to 80 mass % of a nonionic monomer (ex. acrylamide).

## Description

### RELATED APPLICATIONS

This application claims priority to the Japanese Patent Application 2005-30293 dated on February 7, 2005 and is hereby incorporated with reference for all purposes.

### FIELD OF THE INVENTION

This invention relates to a skin cleansing composition, especially the improvement of lather quality at cleansing, moist and soft feeling on the skin after cleansing.

### BACKGROUND OF THE INVENTION

Important point in usage texture required for a skin cleansing composition is the quality of lather generated at cleansing in addition to the primary function, detergency. There is a tendency especially that the creamy lather quality with fine air bubbles is favored by consumers. The skin cleansing composition compounded with surfactants having high detergency sometimes dry out the skin excessively due to the high detergency. Thus, giving favorable moist or soft feeling on the skin after cleansing is desirable. For such problems, the usage texture has been adjusted to be suitable by compounding various kinds of oil, polymer, etc. in the base of skin cleanser.

As some techniques of compounding polymers in the base of skin cleanser, for example, to formulate with a poly (dimethyldiallylammonium chloride) or a dimethyldiallylammonium chloride/acrylamide copolymer (see Patent literature 1, for example), an acrylic acid/ dimethyldiallylammonium chloride/acrylamide copolymer (see Patent literatures 2 and 3, for example), etc. in the skin cleanser are known. However, although these skin cleansers formulated with polymers have improved lathering, they have not been complete in lather quality yet, and it is difficult to say that sufficient effect has been obtained regarding moist or soft feeling to the skin after cleansing. In addition, although excellent conditioning effect of hair cosmetics compounded with acrylic acid/dimethyldiallylammonium chloride/acrylamide terpolymer are known (see Patent literatures 4 and 5, for example), the case of skin cleansers applied with these polymers was not studied.

Patent literature 1 :Japanese unexamined patent publication No. S62-4799
Patent literature 2 :Japanese unexamined patent publication No.2001-64678
Patent literature 3 :Japanese unexamined patent publication No. 2003-73257
Patent literature 4 :United States patent No. 5,296,218
Patent literature 5 :United States patent No. 5,609,862

### DISCLOSURE OF THE INVENTION

### PROBLEMS TO BE SOLVED BY THE INVENTION

This invention was carried out in view of the above-mentioned problems of prior art , and the purpose was to provide a skin cleanser having superior lather quality at cleansing and improved moist and soft feeling after cleansing.

### MEANS FOR SOLVING PROBLEMS

The inventors studied eagerly and, as a result, found that a skin cleanser having extremely superior lather quality at cleansing and extremely improved moist and soft feeling after cleansing could be obtained in the case of compounding amphoteric terpolymer consisting of acrylic acid/dimethyldiallylammonium chloride/ acrylamide which is adjusted each monomers thereof to a specific ratio, and finally completed this invention.

Namely, the skin cleansing composition of this invention is characterized by comprising an amphoteric terpolymer which consists of, 5 to 15 mass % of an anionic monomer shown by the following general formula (1), 15 to 40 mass % of a cationic monomer shown by the following general formula (2), and 45 to 80 mass % of a nonionic monomer shown by the following general formula (3). 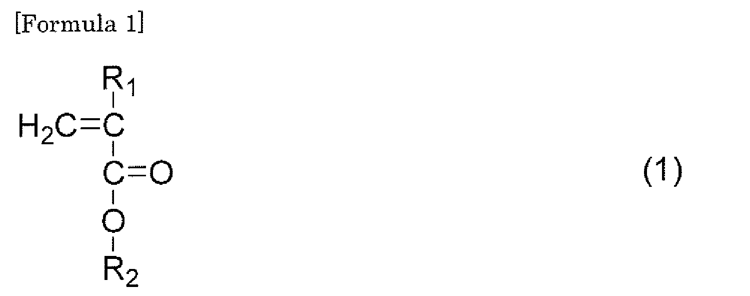 (In the above formula, R₁ means hydrogen or methyl group, and R₂ means hydrogen or monovalent inorganic or organic cation.) 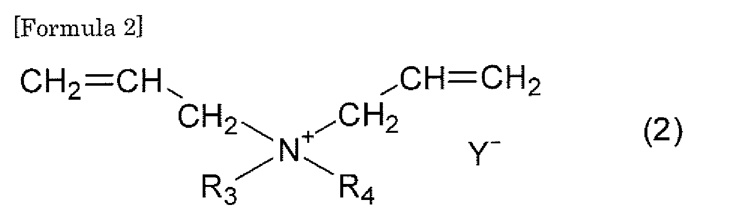 (In the above formula, R₃ and R₄ mean hydrogen or alkyl group having 1 to 4 of carbon atoms, and Y⁻ means monovalent inorganic or organic anion.) 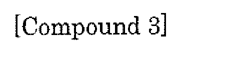 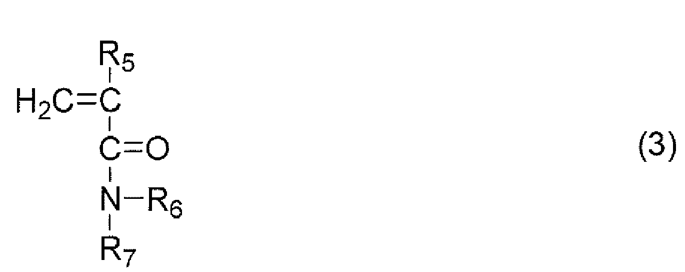 (In the above formula, R₅ means hydrogen or methyl group, R₆ and R₇ mean hydrogen, alkyl group having 1 to 4 of carbon atoms, cycloalkyl group having 3 to 6 of carbon atoms, phenyl group or -(CH₂CH₂O)ₙR₈ group. (n means an integer of 1 to 50, and R₈ means hydrogen, alkyl group having 1 to 4 of carbon atoms, cycloalkyl group having 3 to 6 of carbon atoms or phenyl group))

It is favorable to the skin cleansing composition that the anionic monomer is acrylic acid, the cationic monomer is dimethyldiallylammonium chloride and the nonionic monomer is acrylamide.
It is favorable to the skin cleansing composition that the compounded amount of the amphoteric terpolymer is 0.01 to 5 mass % of the total composition.
It is favorable to the skin cleansing composition that the composition further comprises a fatty acid soap.

### EFFECT OF THE INVENTION

The skin cleansing composition of this invention has been extremely improved in lather quality at cleansing and superior in moist and soft feeling after cleansing due to be compounded the amphoteric terpolymer consisting of anionic monomer/cationic monomer/nonionic monomer adjusted each monomers thereof to a specific ratio.

### BEST MODE FOR CARRYING OUT THE INVENTION

Followings are detailed explanation about favorable mode for carrying out of the invention.
The amphoteric terpolymer used in the skin cleansing composition of this invention consists of, 5 to 15 mass % of anionic monomer expressed by the above general formula (1), 15 to 40 mass % of cationic monomer expressed by the above general formula (2) and 45 to 80 mass % of nonionic monomer expressed by the above general formula (3).

### Anionic monomer

The anionic monomer expressed by the above general formula (1) is either acrylic acid or methacrylic acid, or their salt. In general formula (1), R₁ that is the substituent of α-carbon of acrylic acid is hydrogen or methyl group. Also, R₂ that is the terminal substituent of acrylic acid is hydrogen or monovalent inorganic or organic cation. Any monovalent inorganic or organic cation can be used if it can form a carbonate. Monovalent inorganic cation can be exemplified by sodium ion, potassium ion and lithium ion, and monovalent organic cation can be exemplified by ammonium ion, monoethanolammonium ion and triethanolammonium ion.

Anionic monomer used in this invention can be exemplified by acrylic acid, methacrylic acid, sodium acrylate, sodium methacrylate, ammonium acrylate and ammonium methacrylate. Especially, acrylic acid can be used favorably among these anionic monomers.

The content of the anionic monomer in the amphoteric terpolymer of this invention is 5 to 15 mass % of all component monomers. If the content of the anionic monomer is less than 5 mass % or more than 15 mass % of all component monomers, no good lather quality is available and the moist and soft feeling are not satisfactory when compounded in a skin cleanser. In particular, the content of anionic monomer of 8 to 13 mass % is favorable.

### Cationic monomer

The cationic monomer expressed by the above general formula (2) is diallylammonium salt. In general formula (2), R₃ and R₄ that are the substituent of quaternary ammonium nitrogen are hydrogen or alkyl group having 1 to 4 of carbon atoms. The alkyl group having 1 to 4 of carbon atoms can be either linear or branched, and hydroxyl group or fluorine molecule can substitute for a part. Alkyl group having 1 to 4 of carbon atoms can be exemplified by methyl group, ethyl group, propyl group, butyl group, 2-hydroxylethyl group, 2-hydroxypropyl group, trifluoromethyl group and trifluoroethyl group. In general formula (2), Y⁻ is monovalent inorganic or organic anion, and any one that can form a quaternary ammonium salt can be used. Monovalent inorganic anion can be exemplified by chloride ion, fluoride ion and iodide ion, and monovalent organic anion can be exemplified by sulfate ion, acetate ion, benzensulfonate ion and phosphate ion. R₃ and R₄ can be the same or different.

Cationic monomer used in this invention can be exemplified by dimethyldiallylammonium chloride, diethyldiallylammonium chloride, dipropyldiallylammonium chloride, dimethyldiallylammonium sulfate, diethyldiallylammonium sulfate and dipropyldiallylammonium sulfate. Especially, dimethyldiallylammonium chloride can be used favorably among these cationic monomers.

The content of the cationic monomer in the amphoteric terpolymer of this invention is 15 to 40 mass % of all component monomers. If the content of the cationic monomer is less than 15 mass % or more than 45 mass % of all component monomers, no good lather quality is available and the moist and soft feeling are not satisfactory when compounded in a skin cleanser. In particular, the content of cationic monomer of 17 to 27 mass % is favorable.

### Nonionic monomer

The nonionic monomer expressed by the above general formula (3) is either acrylamide or methacrylamide, or their N-substituted compound. In general formula (3), R₅ that is the substituent of α-carbon of acrylamide is hydrogen or methyl group. Also, in general formula (3), R₆ and R₇ that are the substituent of amide nitrogen are hydrogen, alkyl group having 1 to 4 of carbon atoms, cycloalkyl having 3 to 6 of carbon atoms, phenyl group or -(CH₂CH₂O)ₙR₈ group (n means an integer of 1 to 50, and R₈ means hydrogen, alkyl group having 1 to 4 of carbon, cycloalkyl group having 3 to 6 of carbon atoms or phenyl group). The alkyl group having 1 to 4 of carbon atoms can be either linear or branched, and hydroxyl group or fluorine molecule can substitute for a part. Alkyl group having 1 to 4 of carbon atoms can be exemplified by methyl group, ethyl group, propyl group, butyl group, 2-hydroxylethyl group, 2-hydroxypropyl group, trifluoromethyl group and trifluoroethyl group. In addition, cycloalkyl group having 3 to 6 of carbon atoms can be exemplified by cyclopropyl group, cyclobutyl group, cyclopentyl group and cyclohexyl group. Also, they can be polyethylene glycol group expressed by -(CH₂CH₂O)ₙR₈ group, where n indicating additional mole number of polyethylene glycol is an integer of 1 to 50, and R₈ meaning terminal functional group can be hydrogen, alkyl group having 1 to 4 of carbon atoms, cycloalkyl group having 3 to 6 of carbon atoms or phenyl group. R₆ and R₇ can be the same or different.

Nonionic monomer used in this invention can be exemplified by acrylamide, methacrylamide, N-methylacrylamide, N-methylmethacrylamide, N,N-diethylacrylamide, N,N-diethylmethacrylamide, N-cyclohexylacrylamide, N-cyclohexylmethacrylamide, N,N-di(ethylene glycol)acrylamide, N,N-di(ethylene glycol)methacrylamide, N-polyethylene glycol (10 mole) acrylamide, N-polyethylene glycol (10 mole) methacrylamide, N-polyethylene glycol ethyl ether (10 mole) acrylamide and N-polyethylene glycol ethyl ether (10 mole) methacrylamide. Especially, acrylamide can be used favorably among these nonionic monomers.

The content of above nonionic monomer in the amphoteric terpolymer of this invention is 45 to 80 mass % of all component monomers. If the content of the nonionic monomer is less than 45 mass % of all component monomers, the content of the anionic monomer or the cationic monomer increases relatively, and on the other hand if the content of the nonionic monomer exceeds 80 mass %, the content of the anionic monomer or the cationic monomer decreases relatively, and that leads to the situation that no good lather quality is available and the moist and soft feeling are not satisfactory when compounded in a skin cleanser. In particular, the content of nonionic monomer of 60 to 75 mass % is favorable.

The amphoteric terpolymer used in this invention can be prepared by polymerizing according to publicly known polymerization methods using; 5 to 15 mass % of the anionic monomer, 15 to 40 mass % of the cationic monomer and 45 to 80 mass % of the nonionic monomer. Usable polymerization methods include homogeneous solution polymerization, heterogeneous solution polymerization, emulsion polymerization, reversed phase emulsion polymerization, block polymerization, suspension polymerization and precipitation polymerization. For example, in case of homogeneous solution polymerization the amphoteric terpolymer of the invention can be obtained by dissolving the above each monomers in the solvent in an appropriate monomer composition, adding radical polymerization initiator and heating while stirring in nitrogen atmosphere.

Any solvent can be used for polymerization as long as it can dissolve or suspend each monomers, for example, a water, alcohol-type solvents including methanol, ethanol, propyl alcohol, isopropyl alcohol and butyl alcohol; hydrocarbon-type solvents including hexane, heptane, octane, isooctane, decane and liquid paraffin; ether-type solvents including dimethyl ether, diethyl ether and tetrahydrofuran; ketone-type solvents including acetone and methyl ethyl ketone; ester-type solvents including methyl acetate, ethyl acetate and butyl acetate; chloride-type solvents including methylene chloride, chloroform and carbon tetrachloride, dimethyl formamide, diethyl formamide, dimethyl sulfoxide, dioxane, etc. can be cited. As favorable solvent in particular, water can be used. These solvents can be used as a mixture of two or more. It is generally favorable to select a solvent having higher boiling point than the initiation temperature of employed polymerization initiator.

The polymerization initiator can be selected without limitation from those with the ability to initiate radical polymerization. For example, peroxides including benzoyl peroxide, azo compounds including azobisisobutyronitrile (AIBN) and 2,2'-azobis(isobutyrate), as well as peroxosulfate polymerization initiators including potassium peroxosulfate and ammonium peroxosulfate can be exemplified. However, polymerization can be conducted by photochemical reaction, radiation, etc. without using such polymerization initiators. Polymerization temperature should be at the temperature of initiation of polymerization or higher. For example, a temperature of about 50 to 55°C is enough in general for peroxide polymerization initiators.

Polymerization time is not especially limited, but about 2 hours in general. If polymer with relatively high molecular weight is required, it is desirable to keep reaction for about 1 day. Too short reaction time leaves unreacted monomers, and the molecular weight could become relatively low. Although the average molecular weight of the amphoteric terpolymer used in this invention is not limited particularly, average molecular weight of about 1,300,000 is especially preferable. Although the amphoteric terpolymer used in this invention does not require the order of addition of each monomers in particular, and either block addition or random addition is acceptable, the amphoteric terpolymer added with each monomers randomly is obtained in general.

A representative example of the amphoteric terpolymer (acrylic acid/dimethyldiallylammonium chloride/ acrylamide terpolymer) used in this invention is shown in below general formula (4).

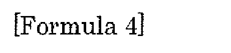

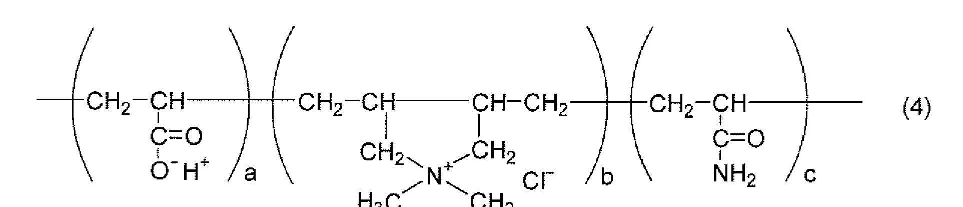

In the above general formula (4), a, b and c means the molar ratio of the anionic monomer (acrylic acid), the cationic monomer (dimethyldiallylammonium chloride) and the nonionic monomer (acrylamide) in all of the component monomers, respectively. For the above amphoteric terpolymer, a, b and c are adjusted to that the anionic monomer is 5 to 15 mass %, the cationic monomer is 15 to 40 mass % and the nonionic monomer is 45 to 80 mass % against all of the component monomers.

The amphoteric terpolymer used in skin cleansing composition of this invention is adjusted to that the anionic monomer is 5 to 15 mass %, the cationic monomer is 15 to 40 mass % and the nonionic monomer is 45 to 80 mass % against all of the component monomers.
Ordinary, general amphoteric terpolymers obtained as showing various properties from anionic to cationic according to the ratio of each constituent monomers. Therefore, the ratio of each monomers in amphoteric terpolymers plays an important role in determining their properties. Namely, the invention gives the specific effect, superior in lather quality at cleansing and improving the moist and soft feeling after cleansing extremely, by formulating the amphoteric terpolymer in which each constituent monomers are adjusted to specific ratio as mentioned above.

The skin cleansing composition of this invention does not limit the compounded amount of the amphoteric terpolymer to be compounded, and the compounded amount can be adjusted for use as appropriate. However, 0.01 to 5 mass %, furthermore 0.1 to 1 mass % against the whole amount of composition is favorable. Less than 0.01 mass % of amphoteric polymer sometimes causes insufficient improvement of lather quality and moist feeling on skin, and the case more than 5 mass % might give sticky feeling.

Furthermore, a surfactant can be incorporated in the skin cleanser of this invention in addition to the above amphoteric terpolymer. The types of surfactants used in this invention are not limited in particular, any one of anionic surfactants, cationic surfactants, amphoteric surfactants and nonionic surfactants can be used, but using fatty acid soap is favorable. Fatty acid soaps can be exemplified by sodium laurate, sodium myristate, sodium palmitate, sodium stearate, potassium laurate, potassium myristate, potassium palmitate and potassium stearate.

Anionic surfactants used in this invention other than the above fatty acid soaps can be exemplified by salt of higher alkyl sulfate esters (e.g., sodium lauryl sulfate and potassium lauryl sulfate); salts of alkyl ether sulfate ester (e.g., triethanolamine POE-lauryl sulfate and sodium POE-lauryl sulfate); N-acylsarcosinic acid (e.g., sodium lauroylsarcosinate); higher fatty acid amide sulfonate salts (e.g., sodium N-myristoyl N-methyltaurine, sodium coconut fatty acid methyltaurine and sodium lauryl methyltaurine); phosphate ester salts(e.g., sodium POE-oleyl ether phosphate and sodium POE-stearyl ether phosphate); sulfosuccinate (e.g., sodium di-2-ethylhexylsulfosuccinate, sodium monolauroylmonoethanolamide polyoxyethylene sulfosuccinate and sodium laurylpolypropylene glycol sulfosuccinate); alkylbenzenesulfonates (e.g., sodium linear dodecylbenzenesulfonate, triethanolamine linear dodecylbenzenesulfonate and linear dodecylbenzenesulfonic acid); higher fatty acid ester sulfate ester salts (e.g., sodium hydrogenated coconut oil fatty acid glycerin sulfate); N-acylglutamate salts (e.g., monosodium N-lauroylglutamate, disodium N-stearoylglutamate and monosodium N-myristoyl-L-glutamate); sulfonated oils (e.g., Turkey red oil); POE-alkyl ether carboxylic acids; POE-alkylallyl ether carboxylic acid salts; α-olefin sulfonate salts; higher fatty acid ester sulfonate salts; secondary alcohol sulfate ester salts; higher fatty acid alkylolamide sulfate ester salts: sodium lauroyl monoethanolamide succinate; ditriethanolamine N-palmitoylaspartate; casein sodium.

Cationic surfactants can be exemplified by alkyltrimethylammonium salts (e.g., stearyltrimethylammonium chloride and lauryltrimethylammonium chloride), alkylpyridinium salts (e.g., cetylpyridinium chloride); distearyldimethylammonium chloride; dialkyldimethylammonium salts; poly (N,N'-dimethyl-3,5-methylenepiperidinium) chloride; alkyl-quat.-ammonium salts; alkyldimethylbenzylammonium salts; alkylisoquinolinium salts; dialkylmorphonium salts; POE-alkylamines; alkylamine salts; polyamine fatty acid derivatives; amyl alcohol fatty acid derivatives; benzalkonium chloride; benzethonium chloride.

Amphoteric surfactants can be exemplified by imidazoline-series amphoteric surfactants (e.g., sodium 2-undecyl-N,N,N-(hydroxyethylcarboxymethyl)-2-imidazolate and 2-cocoyl-2-imidazoliniumhydroxide-1-carboxyethyloxy disodium salts); betaine-series surface active agents (e.g., 2-heptadecyl-N-carboxymethyl-N-hydroxyethylimidazolinium betaine, lauryldimethylaminoacetate betaine, alkylbetaine, amidebetaine and sulfobetaine).

Lipophilic nonionic surfactants can be exemplified by sorbitan fatty acid esters (e.g., sorbitan monooleate, sorbitan monoisostearate, sorbitan monolaurate, sorbitan monopalmitate, sorbitan monostearate, sorbitan sesquioleate, sorbitan trioleate, penta-2-ethylhexylic acid diglycerolsorbitan and tetra-2-ethylhexylic acid diglycerolsorbitan); glycerin polyglycerin fatty acid esters (e.g., cotton oil fatty acid monoglyceride, glyceryl monoerucate, glyceryl sesquioleate , glyceryl monostearate, glyceryl α,α'-oleic acid pyroglutamate and glyceryl monostearate malate); propylene glycol fatty acid esters (e.g., propylene glycol monosterate); hydrogenated castor oil derivatives; glycerin alkyl ether.

Hydrophilic nonionic surfactants can be exemplified by POE-sorbitan fatty acid esters (e.g., POE-sorbitan monooleate, POE-sorbitan monostearate, POE-sorbitan monooleate and POE-sorbitan tetraoleate); POE-sorbitol fatty acid esters (e.g., POE-sorbitol monolaurate, POE-sorbitol monooleate, POE-sorbitol pentaoleate and POE-sorbitol monostearate); POE-glycerin fatty acid esters (e.g., POE-glycerin monostearate, POE-glycerin monoisostearate and POE-glycerin triisostearate); POE-fatty acid esters (e.g., POE-monooleate, POE-distearate, POE-monodioleate and ethylene glycol distearate); POE-alkyl ethers (e.g., POE-lauryl ether, POE-oleyl ether, POE-stearyl ether, POE-behenyl ether, POE-2-octyl dodecyl ether and POE-cholestanol ether); Pluronic types (e.g., Pluronic): POE.POP-alkyl ethers (e.g., POE.POP-cetyl ether, POE-POP-2-decyl tetradecyl ether, POE.POP-monobutyl ether, POE.POP-hydrogenated lanolin and POE.POP-glycerin ether); tetraPOE.tetraPOP-ethylenediamine condensates (e.g., Tetronic); POE-(hydrogenated) castor oil derivatives (e.g., POE-castor oil, POE-hydrogenated castor oil, POE-hydrogenated castor oil monoisostearate, POE-hydrogenated castor oil triisostearate, POE-hydrogenated castor oil monopyroglutamic acid monoisostearic acid diester and POE-hydrogenated castor oil maleic acid); POE-beeswax.lanolin derivatives (e.g., POE-sorbitol beeswax); alkanol amide (e.g., coconut oil fatty acid diethanolamide, lauric acid monoethanolamide and fatty acid isopropanolamide); POE-propylene glycol fatty acid ester; POE-alkylamine; POE-fatty acid amide; sucrose fatty acid ester; alkylethoxydimethylamine oxide; trioleylphosphoric acid.

The skin cleanser of this invention can be formulated in addition to the above ingredients with general ingredients used in cosmetics and drugs in the range that do not damage the effect of the invention. Compoundable ingredients can be exemplified by humectants, powdery ingredients, liquid fats and oils, solid fats and oils, waxes, hydrocarbon oils, higher fatty acids, higher alcohols, synthetic ester oils, silicone oils, natural water-soluble polymers, semi-synthetic water-soluble polymers, synthetic water-soluble polymers, thickeners, ultraviolet absorbers, sequestering agent, lower alcohols, polyhydric alcohols, monosaccharides, oligosaccharides, polysaccharides, amino acids, organic amines, polymer emulsions, pH adjusting agents, antioxidants, auxiliary antioxidants, preservatives, antiphlogistic agents, skin-whitening agents, various extracts, activators, blood circulation stimulators, antiseborrheic agents, anti-inflammatory agents.
Formulations are not specified for the skin cleansers of this invention, and any formulations including solution system, solubilization system, emulsion system, powder dispersion system, water-oil double-layer system, water-oil-powder triple-layer system can be employed.

### Example 1

This invention will be explained below in more detail by showing working examples of the invention, but the invention is not restricted by these examples. In some cases of the following examples we abbreviate acrylic acid as AA, dimethyldiallylammonium chloride as DMDAAC and acrylamide as AM.

At first, synthetic method of amphoteric terpolymer of this invention will be explained.

### Amphoteric terpolymer 1

20 g (123 mmol) of dimethyldiallylammonium chloride (cationic monomer) and 70 g (986 mmol) of acrylamide (nonionic monomer) were charged to a glass reactor equipped with stirring. 10 g (139 mmol) of acrylic acid (anionic monomer) was diluted first with deionized water and then added to the reactor with vigorous stirring to give a total monomer concentration of 14-20%. The monomer mixture was adjusted to pH 6.0 to 6.5 with dilute NaOH, heated to 55°C, and purged with nitrogen for 30 minutes. Polymerization was initiated by adding 560 ppm sodium persulfate. After reaching the peak exotherm, additional dilution water and sodium bisulfite were added to scavenge any residual monomer, the object acrylic acid/ dimethyldiallylammonium chloride/acrylamide (10/20/70) terpolymer was obtained.

### Compounding of the amphoteric terpolymer

The inventors, at first, compared the facial cleanser compounded with the amphoteric terpolymer prepared as above synthetic example and the facial cleanser compounded with a usual cation polymer. The evaluation criteria are shown below. The monomer compositions of polymers used in each examples and comparative examples, formulation of facial cleanser, and the evaluation data are shown in Table 1 below. In the following table, the monomer compositions of each polymer are expressed in mass ratio (%) of each monomers, AA, DMDAAC and AM, in total constituent monomers. Furthermore, the compounded amount of each polymer is described on the pure component basis.

### (1) Lather quality at cleansing

The lather quality at cleansing of the facial cleanser of Examples and Comparative examples were field-tested by 10 specialized panelists. The evaluation criteria are as follows.
Score 5: Creamy
Score 4: Slightly creamy
Score 3: Medial
Score 2: Slightly non-creamy
Score 1: Non-creamy

### (2) Moist feeling of skin after cleansing

The moist feeling of skin after cleansing of the facial cleanser of Examples and Comparative examples were field-tested by 10 specialized panelists. The evaluation criteria are as follows.
Score 5: Having moist feeling.
Score 4: Slightly having moist feeling.
Score 3: Medial.
Score 2: Slightly lacking moist feeling.
Score 1: Lacking moist feeling.

### (3) Soft feeling of skin after cleansing

The soft feeling of skin after cleansing of the facial cleanser of Examples and Comparative examples were field-tested by 10 specialized panelists. The evaluation criteria are as follows.
Score 5: Having soft feeling.
Score 4: Slightly having soft feeling.
Score 3: Medial.
Score 2: Slightly lacking soft feeling.
Score 1: Lacking soft feeling.

**[Table 1]**

| | | | | Com.Ex.1 | Com.Ex.2 | Com.Ex.3 | Ex.1 |
|---|---|---|---|---|---|---|---|
| | AA | DMDAAC | AM | | | | |
| Cationic polymer^{*1} | - | 100.0 | - | - | 0.5 | - | - |
| Cationic copolymer^{*2} | - | 50.0 | 50.0 | - | - | 0.5 | - |
| Amphoteric terpolymer 1 | 10.0 | 20.0 | 70.0 | - | - | - | 0.5 |
| Glycerin | | | | 15.0 | 15.0 | 15.0 | 15.0 |
| Sorbitol solution | | | | 20.0 | 20.0 | 20.0 | 20.0 |
| Polyethylene glycol | | | | 5.0 | 5.0 | 5.0 | 5.0 |
| Lauric acid | | | | 5.0 | 5.0 | 5.0 | 5.0 |
| Myristic acid | | | | 12.0 | 12.0 | 12.0 | 12.0 |
| Palmitic acid | | | | 10.0 | 10.0 | 10.0 | 10.0 |
| Stearic acid | | | | 10.0 | 10.0 | 10.0 | 10.0 |
| Potassium hydroxide | | | | 6.0 | 6.0 | 6.0 | 6.0 |
| Ion-exchange water | | | | Balance | Balance | Balance | Balance |
| (1) Lather quality at cleansing | | | | 2.1 | 2.6 | 3.2 | 4.5 |
| (2) Moist feeling of skin after cleansing | | | | 2.0 | 2.6 | 3.6 | 4.6 |
| (3) Soft feeling of skin after cleansing | | | | 2.0 | 2.6 | 3.3 | 4.6 |

| | | | | | | | |
|---|---|---|---|---|---|---|---|
| *1: Merquat 100 (Nalco) *2: Merquat 2200 (Nalco) | | | | | | | |

As shown in Table 1 above, the facial cleanser of Comparative Example 1 compounded with no polymers was inferior in lather quality at cleansing, and neither moist feeling nor soft feeling of skin after cleansing was available. Meanwhile, in Comparative Example 2 compounded with poly (dimethyldiallylammonium chloride) as cationic polymer, and in Comparative Example 3 compounded with dimethyldiallylammonium chloride/ acrylamide copolymer as cationic polymer, all lather quality, moist and soft feeling were improved a little, but it is difficult to say that satisfactory effect has been obtained.
On the contrary, the facial cleanser of Example 1 compounded with the amphoteric terpolymer 1 consisting of acrylic acid/ dimethyldiallylammonium chloride/ acrylamide was extremely superior in lather quality at cleansing, and apparently the moist and soft feeling of the skin after cleansing were improved extremely.

### Monomer composition of amphoteric terpolymer

Subsequently, the inventors evaluated the facial cleanser compounded with various amphoteric terpolymers with different ratio of monomer in order to study the favorable monomer composition of amphoteric terpolymer. The monomer compositions of amphoteric terpolymers used in each Example and Comparative Examples, formulation of facial cleanser, and the evaluation data are shown in Table 2 below. The evaluation criteria are the same as the previous test.

**[Table 2]**

| | | | | Com.Ex.4 | Com.Ex.5 | Com.Ex.6 | Ex. 2 | Ex.3 |
|---|---|---|---|---|---|---|---|---|
| | AA | DMDAAC | AM | | | | | |
| Amphoteric terpolymer 2^{*1} | 20 | 80 | - | 0.5 | - | - | - | - |
| Amphoteric terpolymer 3^{*2} | 25 | 50 | 25 | - | 0.5 | - | - | - |
| Amphoteric terpolymer 4^{*3} | 17 | 45 | 38 | - | - | 0.5 | - | - |
| Amphoteric terpolymer 5 | 15 | 15 | 70 | - | - | - | 0.5 | - |
| Amphoteric terpolymer 6 | 5 | 40 | 55 | - | - | - | - | 0.5 |
| Glycerin | | | | 15.0 | 15.0 | 1.5.0 | 15.0 | 15.0 |
| Sorbitol solution | | | | 20.0 | 20.0 | 20.0 | 20.0 | 20.0 |
| Polyethylene glycol | | | | 5.0 | 5.0 | 5.0 | 5.0 | 5.0 |
| Lauric acid | | | | 5.0 | 5.0 | 5.0 | 5.0 | 5.0 |
| Myristic acid | | | | 12.0 | 12.0 | 12.0 | 12.0 | 12.0 |
| Palmitic acid | | | | 10.0 | 10.0 | 10.0 | 10.0 | 10.0 |
| Stearic acid | | | | 10.0 | 10.0 | 10.0 | 10.0 | 10.0 |
| Potassium hydroxide | | | | 6.0 | 6.0 | 6.0 | 6.0 | 6.0 |
| Ion-exchange water | | | | Balance | Balance | Balance | Balance | Balance |
| (1) Lather quality at cleansing | | | | 3.2 | 3.6 | 3.6 | 4.2 | 4.0 |
| (2) Moist feeling of skin after cleansing | | | | 3.4 | 2.4 | 2.4 | 4.4 | 4.2 |
| (3) Soft feeling of skin after cleansing | | | | 3.3 | 2.2 | 2.2 | 4.2 | 4.2 |

| | | | | | | | | |
|---|---|---|---|---|---|---|---|---|
| *1: Merquat 280 (Nalco) *2: Merquat Plus 3300 (Nalco) *3: Merquat Plus 3331 (Nalco) | | | | | | | | |

As shown in Table 2 above, Comparative Example 4 compounded with the amphoteric terpolymer not containing acrylamide was a little improved in all of the lather quality, moist and soft feeling when compared with Comparative Example 1 without compounding of polymers, but it is difficult to say that sufficient effect has been obtained. Also, Comparative Example 5 and 6 compounded with the amphoteric terpolymer 3 or 4 containing 17 to 25% of acrylic acid and 45 to 50% of dimethyldiallylammonium chloride did not have sufficient lather quality, moist and soft feeling.
On the contrary, the facial cleanser of Examples 2 and 3 compounded with the amphoteric terpolymer 5 or 6 containing 5 to 15% of acrylic acid and 15 to 40% of dimethyldiallylammonium chloride were superior in lather quality, and apparently the moist and soft feeling were improved significantly.
The above results indicate that the skin cleanser of this invention require the ratio of anionic monomer of 5 to 15 mass % and that of cationic monomer of 15 to 40% in the amphoteric terpolymer.

### Compounded amount of amphoteric terpolymer in skin cleanser

Subsequently, the inventors prepared and evaluated the facial cleanser compounded with various compounded amount of the amphoteric terpolymer in order to study the favorable concentration of amphoteric terpolymers in the formulation of skin cleansing composition. The formulations of facial cleanser of each example and the evaluation data are shown in Table 3 below. The evaluation criteria are the same as the previous test.

**[Table 3]**

| | Ex.4 | Ex.5 | Ex.1 | Ex.6 | Ex.7 |
|---|---|---|---|---|---|
| Amphoteric terpolymer 1 | 0.01 | 0.1 | 0.5 | 1.0 | 5.0 |
| Glycerin | 15.0 | 15.0 | 15.0 | 15.0 | 15.0 |
| Sorbitol solution | 20.0 | 20.0 | 20.0 | 20.0 | 20.0 |
| Polyethylene glycol | 5.0 | 5.0 | 5.0 | 5.0 | 5.0 |
| Lauric acid | 5.0 | 5.0 | 5.0 | 5.0 | 5.0 |
| Myristic acid | 12.0 | 12.0 | 12.0 | 12.0 | 12.0 |
| Palmitic acid | 10.0 | 10.0 | 10.0 | 10.0 | 10.0 |
| Stearic acid | 10.0 | 10.0 | 10.0 | 10.0 | 10.0 |
| Potassium hydroxide | 6.0 | 6.0 | 6.0 | 6.0 | 6.0 |
| Ion-exchange water | Balance | Balance | Balance | Balance | Balance |
| (1) Lather quality at cleansing | 4.0 | 4.2 | 4.5 | 4.7 | 4.7 |
| (2) Moist feeling of skin after cleansing | 4.0 | 4.4 | 4.6 | 4.8 | 4.8 |
| (3) Soft feeling of skin after cleansing | 4.2 | 4.4 | 4.6 | 4.7 | 4.7 |

As shown in Table 3 above, Examples 1 and 4 to 7 compounded with 0.01 to 5.0% of the amphoteric terpolymer showed the effect of improvement of the lather quality, moist and soft feeling is seen. From the above result compounding 0.01 to 5mass% of the amphoteric terpolymer to the skin cleansers of this invention is favorable.

Other examples of this invention are described below, but this invention is not restricted by these examples.

### Amphoteric terpolymer 7

20 g (123 mmol) of diethyldiallylammonium chloride (cationic monomer) and 70 g (986 mmol) of acrylamide (nonionic monomer) were charged to a glass reactor equipped with stirring. 10 g (139 mmol) of acrylic acid (anionic monomer) was diluted first with deionized water and then added to the reactor with vigorous stirring to give a total monomer concentration of 1.4-20%. The monomer mixture was adjusted to pH 6.0 to 6.5 with dilute NaOH, heated to 55°C, and purged with nitrogen for 30 minutes. Polymerization was initiated by adding 560 ppm sodium persulfate. After reaching the peak exotherm, additional dilution water and sodium bisulfite were added to scavenge any residual monomer, the object acrylic acid/ diethyldiallylammonium chloride/ acrylamide terpolymer was obtained.

### Amphoteric terpolymer 8

20 g (123 mmol) of diethyldiallylammonium chloride (cationic monomer) and 70 g (986 mmol) of acrylamide (nonionic monomer) were charged to a glass reactor equipped with stirring. 10 g (139 mmol) of methacrylic acid (anionic monomer) was diluted first with deionized water and then added to the reactor with vigorous stirring to give a total monomer concentration of 14-20%. The monomer mixture was adjusted to pH 6.0 to 6.5 with dilute NaOH, heated to 55°C, and purged with nitrogen for 30 minutes. Polymerization was initiated by adding 560 ppm sodium persulfate. After reaching the peak exotherm, additional dilution water and sodium bisulfite were added to scavenge any residual monomer, the object methacrylic acid/ dimethyldiallylammonium chloride/ acrylamide terpolymer was obtained.

**[Table4]**

| Example 8 Facial cleanser | Compounded amount (mass %) |
|---|---|
| Amphoteric terpolymer 7 | 0.5 |
| Glycerin | 15.0 |
| Polyethylene glycol 400 | 5.0 |
| Lauric acid | 5.0 |
| Myristic acid | 10.0 |
| Palmitic acid | 10.0 |
| Stearic acid | 15.0 |
| Sodium lauroylmethyltaurine | 5.0 |
| Fatty acid monoglyceride | 1.0 |
| Polyglyceryl monolaurate | 1.0 |
| Potassium hydroxide | 6.0 |
| Ion-exchange water | Balance |
| Perfume | q.s. |

(Manufacturing method) Glycerin, polyethylene glycol 400, lauric acid, myristic acid, palmitic acid, stearic acid and fatty acid monoglyceride were added to ion-exchange water and dissolved by heating at 75°C. After dissolution, the mixture was neutralized with potassium hydroxide solution, then the amphoteric polymer and residual ingredients were added, mixed thoroughly and cooled, and the facial cleanser was obtained.
The facial cleanser of Example 8 was superior extremely in the moist feeling and soft feeling of the skin after cleansing together with the lather quality at cleansing.

**[Table5]**

| Example 9 Body shampoo | Compounded amount (mass %) |
|---|---|
| Amphoteric terpolymer 8 | 0.5 |
| Glycerin | 15.0 |
| Polyethylene glycol 400 | 5.0 |
| Lauric acid | 2.0 |
| Myristic acid | 5.0 |
| Palmitic acid | 5.0 |
| Stearic acid | 7.0 |
| Sodium lauroylmethyltaurine | 5.0 |
| Sodium laurylglycolacetate | 1.0 |
| Potassium hydroxide | 3.0 |
| Cationated guar gum | 0.2 |
| Ion-exchange water | Balance |
| Perfume | q.s. |

(Manufacturing method) Glycerin, polyethylene glycol 400, lauric acid, myristic acid, palmitic acid and stearic acid were added to ion-exchange water and dissolved by heating at 75°C. After dissolution, the mixture was neutralized with potassium hydroxide solution, then the polymer and residual ingredients were added, mixed thoroughly and cooled, and the body shampoo was obtained.
The body shampoo of Example 9 was superior extremely in the moist and soft feeling of the skin after cleansing together with the lather quality at cleansing.

## Claims

1. A skin cleansing composition comprising an amphoteric terpolymer which consists of;
5 to 15 mass % of an anionic monomer shown by the following general formula (1),
15 to 40 mass % of a cationic monomer shown by the following general formula (2) and
45 to 80 mass % of a nonionic monomer shown by the following general formula (3). 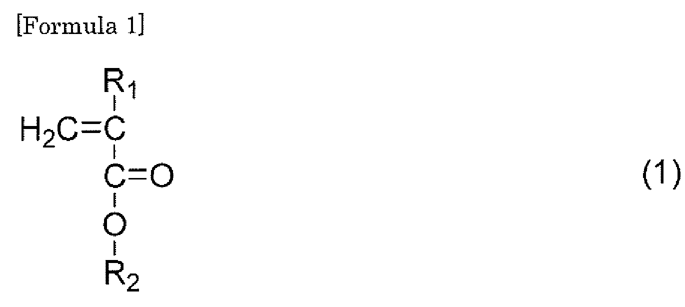 (In the above formula, R₁ means hydrogen or methyl group, and R₂ means hydrogen or monovalent inorganic or organic cation.) 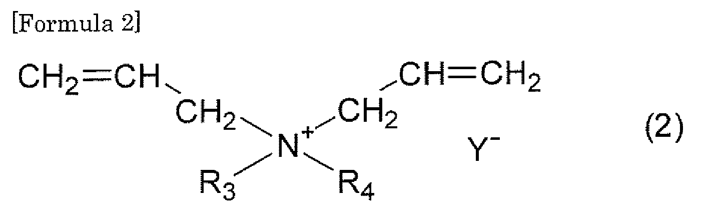 (In the above formula, R₃ and R₄ mean hydrogen or alkyl group having 1 to 4 of carbon atom, and Y- means monovalent inorganic or organic anion.) 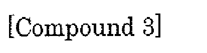 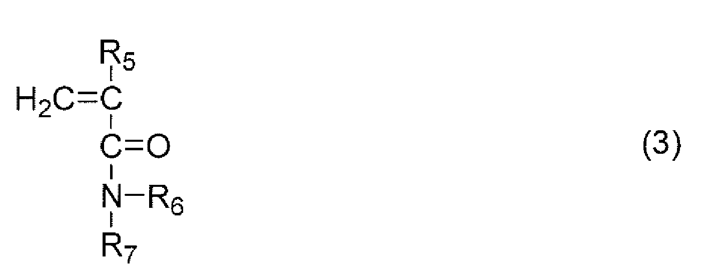 (In the above formula, R₅ means hydrogen or methyl group, R₆ and R₇ mean hydrogen, alkyl group having 1 to 4 of carbon atom, cycloalkyl group having 3 to 6 of carbon atom, phenyl group or -(CH₂CH₂O)ₙR₈ group. (n means an integer of 1 to 50, and R₈ means hydrogen, alkyl group having 1 to 4 of carbon atom, cycloalkyl group having 3 to 6 of carbon atom or phenyl group))

2. The skin cleansing composition according to claim1, wherein the anionic monomer is acrylic acid, the cationic monomer is dimethyldiallylammonium chloride and the nonionic monomer is acrylamide.

3. The skin cleansing composition according to claim 1 or 2, wherein the compounded amount of the amphoteric terpolymer is 0.01 to 5 mass % of the total composition.

4. The skin cleansing composition according to any claim of 1 to 3, wherein the composition further comprises a fatty acid soap
